# EUROPEAN PATENT APPLICATION

(11) **EP 2 495 309 A1**
(43) Date of publication of application: **05.09.2012**
(21) Application number: 10826847.5
(22) Date of filing: 29.10.2010
(51) Int. Cl.: C12N 5/071, A61L 27/00, C12M 3/00, C12N 5/07

(54) **METHOD FOR PREPARATION OF PROLIFERATABLE ANIMAL CELLS**

(30) Priority: 30.10.2009 JP 2009250518
(71) Applicant: Japan Agency for Marine-Earth Science and Technology, Yokosuka-shi Kanagawa 237-0061 (JP)
(72) Inventor: KOYAMA, Sumihiro, Yokosuka-shi Kanagawa 237-0061 (JP)
(74) Representative: Vossius & Partner
(86) International application number: PCT/JP2010/069272
(87) International publication number: WO 2011/052717

(57) **Abstract**

A method for preparing animal cells with good adhesion and proliferation properties which are capable of proliferation is provided. the method permits the detachment of cultured cells without damaging the cells. A method for preparing a sheet of animal cells such as skin cells which are capable of proliferation is also provided. A method for preparing animal cells capable of proliferation, comprising the steps of (1) culturing animal cells on a substrate at least one portion of which is an electrode, and (2) applying a high-frequency wave potential to the electrode to detach the cells that have adhered to the substrate surface through culturing. The high-frequency wave potential is of a frequency falling within a range of 1 KHz to 10 MHz with a potential falling within a range of ± 1.0 V (vs. Ag/AgCl) or less. The culture medium during separation does not contain calcium or magnesium.

## Description

### [Cross-Reference to Related Patent Applications]

The present application claims priority under Japanese Patent Application 2009-250518 filed on October 30, 2009, the contents of the entirety of which are incorporated herein by reference.

### [Technical Field]

The present invention relates to a method for preparing animal cells capable of proliferation by separating animal cells that have been cultured on a substrate from the substrate in a state permitting proliferation following culturing, and to a method for preparing an animal cell sheet utilizing this method. More particularly, the present invention relates to a method for preparing a skin cell sheet utilizing this separation method.

### [Background Art]

The main treatment material in regenerative medicine is human cells provided by the patient or a donor. For a treatment by transplant, it is essential to cause the human cells to proliferate and to ensure a suitable number of cells by cell culturing. Many cells are anchorage-dependent cells that proliferate by a series of steps of adhering to a culture surface, spreading, and dividing. The number of cells can be increased by subculturing the cells several times. In this process, the required elemental techniques consist of a culture surface allowing the cells to adhere, spread, and proliferate, and a control to detach the cells from the culture surface. A surface that not only allows the cells to adhere and proliferate, but also permits separation of the cells in a condition capable of proliferation without damaging the cells that have proliferated is important.

Conventionally known methods of cell separation include the enzymatic method, the electric stimulation method, and the method of controlling the degree of hydrophilicity. In the enzymatic method, the protein on the outer layer of the cells is dissolved with a protease such as trypsin to detach the cells. In the electric stimulation method, an electric current is passed through the culture surface and the protein on the outer layer of the cells is dissolved to detach the cells. In the method of controlling the degree hydrophilicity, the hydrophilic property of the culture surface is increased to detach the cells. Of these methods, the enzymatic method has become the most commonly employed. However, since the enzymatic method dissolves the entire outer layer of the cells, there is considerable damage to the cells. Thus, there is a problem in that re-adhesion and proliferation efficiency is poor.

The electric stimulation method makes it possible to induce a local reaction at the point of contact between the cells and culture surface and achieve a rapid response. For example, Patent Reference 1 describes adjusting the potential of an electrode to which cultured cells have adhered to detach the cells spontaneously, yielding cultured cells with little damage. Patent Reference 2 describes applying a constant potential to an electrode to which cells have adhered to detach the cells.

### [Prior Art References]

### [Patent References]

[Patent Reference 1] Japanese Unexamined Patent Publication (KOKAI) No. 2005-312343
[Patent Reference 2] Japanese Unexamined Patent Publication (KOKAI) Heisei No. 10-42857

The entire contents of Patent References 1 and 2 are incorporated herein by reference.

### [Summary of the Invention]

### [Problem to Be Solved by the Invention]

However, when employing the methods described in Patent References 1 and 2, the animal cells that have adhered to the electrode sometimes fail to be detached. Even when they are detached, there are problems in that the detached cells are damaged, the rate of proliferation is low, and the ability to proliferate is poor. For example, under the conditions described in Patent Reference 2, the cells are detached at a constant potential of -1.2 V (vs. Ag/AgCl). However, hydrogen tends to be produced, damaging the cells and resulting in a low ratio of animal cells capable of proliferation.

Accordingly, one object of the present invention is to provide a method for preparing animal cells that are capable of proliferation permitting the separation of cultured cells with good adhesion and proliferation properties that are not damaged following proliferation. A further object of the present invention is to provide a method for preparing a sheet of animal cells such as skin cells that are capable of proliferation.

### [Means of Solving the Problems]

The present inventors conducted a variety of research resulting in the discovery that the use of a high-frequency wave potential to detach cultured cells from an electrode surface solved the above-stated problems. The present invention was devised on that basis.

The present invention is as follows:
[1] A method for preparing animal cells capable of proliferation, comprising the steps of (1) culturing animal cells on a substrate surface at least one portion of which is an electrode, and (2) applying a high-frequency wave potential to the electrode to detach the cells that have adhered to the substrate surface through culturing, wherein the high-frequency wave potential is of a frequency falling within a range of 1 KHz to 10 MHz and the range of the potential is ± 1.0 V (vs. Ag/AgCl) or less; and the culture medium during the detachment step is a culture mediumcontaining neither Ca²⁺ nor Mg²⁺
[2] The preparation method according to [1], wherein the culture mediumduring the detachment step is a phosphate buffer solution (containing neither Ca²⁺ nor Mg²⁺).
[3] The preparation method according to [1] or [2], wherein the high-frequency wave potential is a rectangular wave, sinusoidal wave, or triangular wave.
[4] The preparation method according to any one of [1] to [3], wherein the entire substrate surface is an electrode.
[5] The preparation method according to any one of [1] to [3], wherein a portion of the substrate surface is an electrode and cells on the electrode surface and on a nonelectrode surface in proximity to the electrode are detached in step (2).
[6] The preparation method according to any one of [1] to [5], further comprising the step of subculturing the animal cells capable of proliferation which have been detached to keep culturing animal cells that are capable of proliferation.
[7] The preparation method according to any one of [1] to [6], wherein the cells are cultured in the form of a sheet, and the sheet of the cells is detached in a condition capable of proliferation to obtain a sheet of animal cells capable of proliferation.
[8] The preparation method according to any one of [1] to [7], wherein the animal cells are skin cells.

### [Effect of the Invention]

Based on the present invention, animal cells that have adhered to an electrode can be readily detached by the application of a high-frequency wave potential to obtain detached cells with a high rate of proliferation and good proliferating ability. The present invention permits the subculturing of stem cells, iPS cells, and the like that present risks such as mutation with chemical subculturing methods.

### [Brief Description of the Drawings]

[Figure 1] Drawings describing steps (1) and (2).
[Figure 2] Drawings describing the electrode fabrication procedure in Reference Example 1.
[Figure 3] Images of electrode surfaces with the application of a voltage for 0, 30, and 60 minutes obtained in Example 1.
[Figure 4] An image of the electrode surface following electric separation after subculturing the cells in a culture bottle.
[Figure 5] Images of the application of a voltage for 0 and 60 minutes obtained in Comparative Example 1.
[Figure 6] Images of the electrode surface after applying a potential of ±1.0 V and 3 MHz to human skin fibroblasts in medium, obtained in Comparative Example 2.
[Figure 7] Images of the electrode surface after applying a constant potential of - 1.0 V (vs. Ag/AgCl) for 0 and 60 minutes, obtained in Comparative Example 3.
[Figure 8] An image of the electrode surface following the subculturing in a culture bottle of cells following electric separation, obtained in Comparative Example 3.
[Figure 9] Images of the electrode surface for voltage application periods of 0, 30, and 60 minutes, obtained in Example 2.
[Figure 10] The results of measurement of the water contact angle of an ITO electrode surface in Reference Example 2.

### [Modes of Carrying Out the Invention]

The present invention relates to a method for preparing animal cells capable of proliferation.
The present invention comprises steps (1) and (2) below.

### Step (1)

Step (1) is a step of culturing animal cells on a substrate surface at least one portion of which is an electrode. The "substrate at least one portion of which is an electrode" on which the animal cells are cultured is not specifically limited. The entire surface of the substrate can be an electrode, or some part thereof can be an electrode and another part thereof can be a nonelectrode (substrate). For example, the substrate at least some portion of which is an electrode can be one in which an electrode layer is formed on a substrate that is not an electrode, or one in which the entire substrate is an electrode, such as a carbon electrode. When an electrode is provided on a substrate that is not an electrode, the substrate can be one in which an electrode is present on part or all of the surface of an insulating substrate. An example of such a substrate on which an electrode is present is an indium [tin] oxide (ITO) coating formed on a glass slide. However, there is no intent to limit the insulating substrate to a glass slide. So long as it is a non-electrically conductive solid, there is no specific limitation. The insulating substrate can be comprised of an electrically nonconductive organic or inorganic material. In addition to glass, examples of organic and inorganic materials that are electrically nonconductive are plastics and ceramics. There is no intent to limit the electrode to indium [tin] oxide (ITO). An electrode comprised of a known electrode material can be suitably employed.

When providing an electrode layer on the substrate, the electrode layer can be provided over the entire substrate surface, or the electrode layer can be provided over part of the surface of an electrode substrate. When an electrode layer is provided over part of the surface of an electrode substrate, the electrode layer can be in the form of an array or stripes, for example. In the method of the present invention, as a particular result of culturing, animal cells in the form of a sheet can be readily detached without being damaged. The electrode layer can be suitably determined taking into account the desired size (surface area and dimensions) of the animal cell sheet. For example, the surface area of the electrode can fall within a range of 1 to 900 cm .

The term "in the form of an array" means, for example, that the electrode layer is arranged as multiple microregions in rows and columns. The number of microregions that are arranged in rows and columns is not specifically limited, and can be suitably determined based on the type (size) of the animal cells and the use objective of the substrate on which the animal cells are arranged in an array. For example, the number can fall within a range of 10 to 10⁵ vertically and 10 to 10⁵ horizontally. However, there is no intent to limit this range. The shape of the electrode layer surfaces can be rectangular (triangular, square, rectangular, polyhedral, or the like), circular, elliptical, or the like, and can be suitably determined. The dimensions of the individual electrode surfaces in the form of an array permit the adhesion of a single animal cell to a single electrode surface surface. The dimensions of the animal cells will vary with the cell; the dimensions of the electrode surfaces can be suitably determined based on the dimensions of the animal cells that are being caused to adhere. However, when the animal cells are HeLa cells, the dimensions of the electrode surfaces can fall within a range of 25 to 100 µm, for example. The dimensions of the individual electrode surfaces in the form of an array can also permit the adhesion of two or more animal cells to each electrode surface. The spacing of the electrodes can, for example, fall within a range of 25 to 100 µm.

An electrode layer in the form of stripes, for example, can be comprised of multiple belt-shaped electrode layers of equal width that are positioned with an equal or unequal spacing, or multiple belt-shaped electrode layers of unequal width that are positioned with an equal or unequal spacing. The width of the belt-shaped electrode layers and the spacing between the belt-shaped electrode layers are not specifically limited. Each can independently fall within a range of 25 to 100 µm, for example.

As set forth further below, in step (2), it is possible to detach not just cells on the electrode surface, but also cells on a nonelectrode surface in the vicinity of the electrode. The distance from the electrode of the cells on the nonelectrode surface that can be detached also depends on the frequency and potential of the high-frequency wave potential, but can be about 100 µm or less. Accordingly, both when in the form of an array and in the form of stripes, an electrode spacing falling within the above range of 25 to 100 µm also permits the separation of cells on the nonelectrode surface.

The electrode layer in the form of an array or stripes can be formed on a substrate surface, for example, by coating an electrode layer on the substrate surface, forming a mask for the electrode surfaces in the form of an array or stripes on the surface of the electrode layer, etching the surface of the electrode layer through the mask, and removing the mask. Alternatively, the electrode surfaces in the form of an array or stripes can be formed on the substrate surface by coating an electrode layer on the substrate surface through a mask for the electrode surfaces in the form of an array or stripes, and then removing the mask. The formation of the electrode layer and etching of the outer surface of the electrode layer can be suitably implemented by the usual methods.

In the present invention, the animal cells that are used to prepare animal cells capable of proliferation are not specifically limited. Examples are skin cells, step cells, and iPS cells.

Suitable known culturing conditions can be adopted based on the type of animal cell as the culturing conditions for the animal cells on the surface of the substrate at least one part of which is an electrode. When a culture medium containing animal cells is provided on the substrate surface at least one part of which is an electrode and the animal cells are cultured under conditions based on the type of animal cell, when both an electrode surface and a nonelectrode surface are present, the animal cells also adhere to the nonelectrode surface. The culturing of animal cells on a substrate surface at least one part of which is an electrode can be conducted under conditions where the animal cells are individually present, or under conditions where the animal cells form a patch. The animal cells can also be cultured under conditions where they form a sheet-like patch. In the present invention, in step (2), both animal cells that form a patch and animal cells that form a sheet-like patch can be detached in a condition capable of proliferating.

### Step (2)

Step (2) is a step in which a high-frequency wave potential is applied to the substrate at least one portion of which is an electrode to detach the cells that have adhered to the substrate surface through culturing. By applying a high-frequency wave potential, the cells that have adhered to the electrode and the cells on the nonelectrode surface in proximity to the electrode are detached. Specifically, the frequency of the high-frequency wave potential falls within a range of 1 KHz to 10 MHz, desirably within a range of 1 to 5 MHz. The potential thereof is, for example, a range of ± 1.0 V (vs. Ag/AgCl) or less, and by way of example, can be made ± 0.9 V (vs. Ag/AgCl), ± 0.8 V (vs. Ag/Agcl), or the like. The waveform of the high-frequency wave potential is, for example, rectangular, sinusoidal, triangular, or the like.

In the course of applying the high-frequency wave potential, the culture medium during separation is suitably a medium not containing calcium or magnesium. That is because the cells was not electrically detached when a high-frequency wave potential was applied to a culture medium containing calcium or magnesium. In the course of applying a high-frequency wave potential, the culture medium during separation can be, for example, a phosphate buffer solution (containing neither Ca²⁺ nor Mg²⁺) (PBS(-)), Hank's buffer solution (containing neither Ca²⁺ nor Mg²⁺), or the like. Of these, PBS(-) is desirable.

In step (2), not just cells on the electrode surface, but also cells on the nonelectrode surface in proximity to the electrode can be detached. The distance from the electrode of the cells on the nonelectrode surface that can be separated depends on both the frequency and potential of the high-frequency wave potential, but is about 100 µm or less. In the course of applying a high-frequency wave potential, the potential is directly applied to the cells on the electrode and indirectly applied to the cells on the nonelectrode surface. Accordingly, the stress on the cells due to the application of the potential is relatively low for the cells on the nonelectrode surface. Even when the cells are damaged by the application of the potential (under such high-frequency wave potential conditions), the cells on the nonelectrode surface tend to be damaged less or not at all. A high-frequency wave potential that is strong enough to damage the cells when applied will sometimes be applied when the cells have attached firmly and are difficult to be detached. In such cases, an electrode shape or arrangement that preferentially detaches the cells on the nonelectrode surface can be employed, or the nonelectrode surface can be selected to actively detach the cells on the nonelectrode surface.

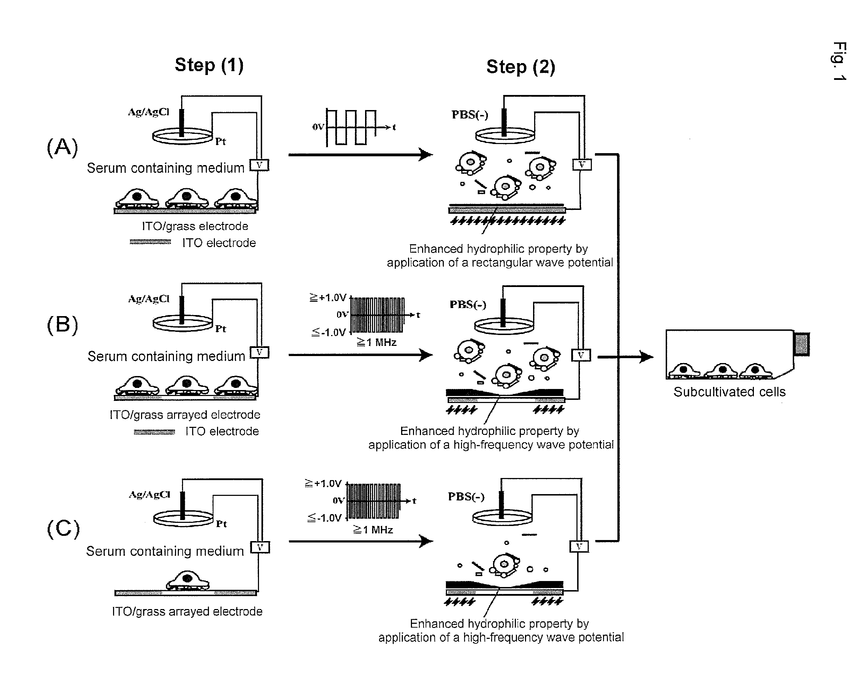

As shown in Figure 1, in the method of the present invention, animal cells are cultured on the electrode surface or on the electrode and nonelectrode surface of the substrate and caused to adhere in step (1). (A) is the case where the entire surface of the substrate is an electrode surface, and (B) and (C) are cases where part of the substrate is an electrode and part is a nonelectrode surface. In all of these cases, the application of the high-frequency wave potential in step (1) causes the animal cells that have attached to the electrode surface or the electrode and nonelectrode surfaces to be detached.

The method of the present invention can further comprise the step of subculturing the animal cells capable of proliferation which have been detached, and maintaining animal cells that are capable of proliferation. A suitable method of maintaining the animal cells can be adopted based on the type of animal cell.

In step (1), animal cells can be cultured on an electrode surface, caused to adhere, and separated in the form of a sheet in a condition in which they are capable of proliferation as a sheet-like patch. This is thus extremely useful in the field of regenerative treatments and the like.

### [Examples]

The present invention is described below in greater detail through examples.

### Reference Example 1

### (1) Fabricating the electrode

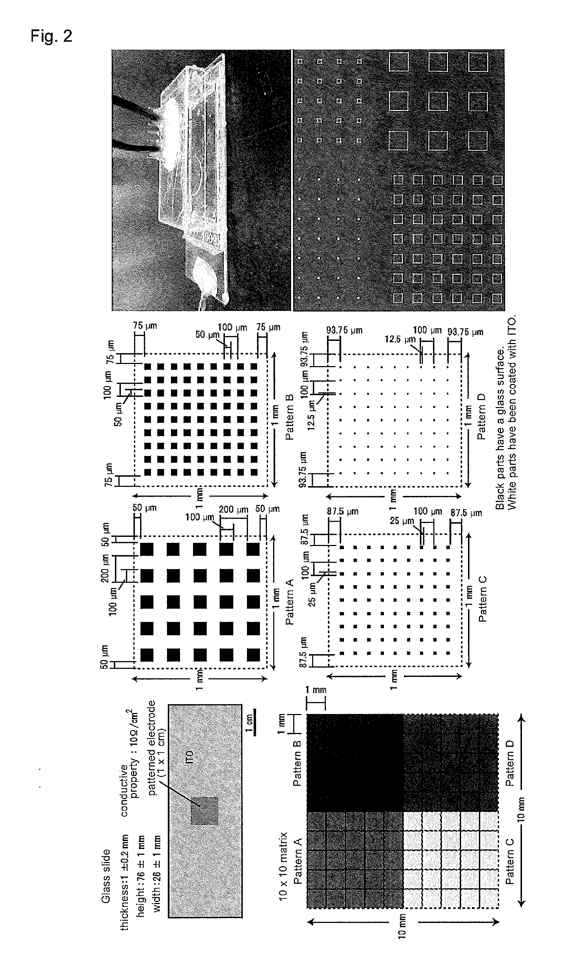

Electrodes and a three-electrode culturing system were fabricated by the procedure given below. Figure 2 gives descriptive drawings of the procedure of fabricating the electrodes.

1) A patterned electrode (1 x 1 cm) coated with indium [tin] oxide (ITO) to 6.3 to 7.5 Ω/cm² in the center portion of a glass slide (upper left in Figure 2) was prepared (lower left in Figure 2). The patterned electrode, as shown on the lower left, was prepared with four separate regions. The patterns A to D of the individual regions are shown in the center of Figure 2. The black portions denote the glass surface, and the white portions denote the ITO electrode surface. A patterned electrode of this form was employed in the examples.
2) Using a diamond drill, holes were opened in the edge portion of the glass slide and connections were made between the wiring and the ITO electrode with an electrically conductive resin (Dotite, D-550, Fujikura Kasei (Ltd.)).
3) The chamber portion of a plastic chamber slide (Lab-tek chamber slide, 177410, NalgeNunc) was removed and a silicon bonding agent (Toshiba Silicone, TSE382-C clear), used for repairing aquariums, was employed to bond it onto the ITO patterned electrode.
4) Holes were opened in two spots in the cover portion of the plastic chamber slide using a diamond drill, and connections were made with a platinum electrode and a silver/silver chloride electrode.
5) The cover and chamber were combined to complete a three-electrode culture system (photograph in upper right).

### Example 1

Animal cells were electrically subcultured by the following procedure using the three-electrode culture system fabricated in Reference Example 1.

1) Animal cells (normal human skin fibroblasts) suspended in a serum-containing medium were inoculated into the ITO patterned electrode chamber of the three-electrode culture system and cultured for several days.
2) Attachment of the cells to the electrode substrate was confirmed and the interior of the ITO patterned electrode chamber was replaced with PBS(-).
3) A potentiostat (PS-14, Toho Technical Research) connected to a function generator (AD8624A, A&D Company, Tokyo, Japan) was used to apply a rectangular waveform wave potential of 3 MHz, ± 1.0 V (vs. Ag/AgCl) to the ITO patterned elected in the three-electrode culture system.
4) The cells detached from the electrode were recovered and transferred to a fresh culture bottle. The cell adhesion rate and presence or absence of cellular proliferation were then observed by phase-contrast microscopy (CKX-41, Olympus).

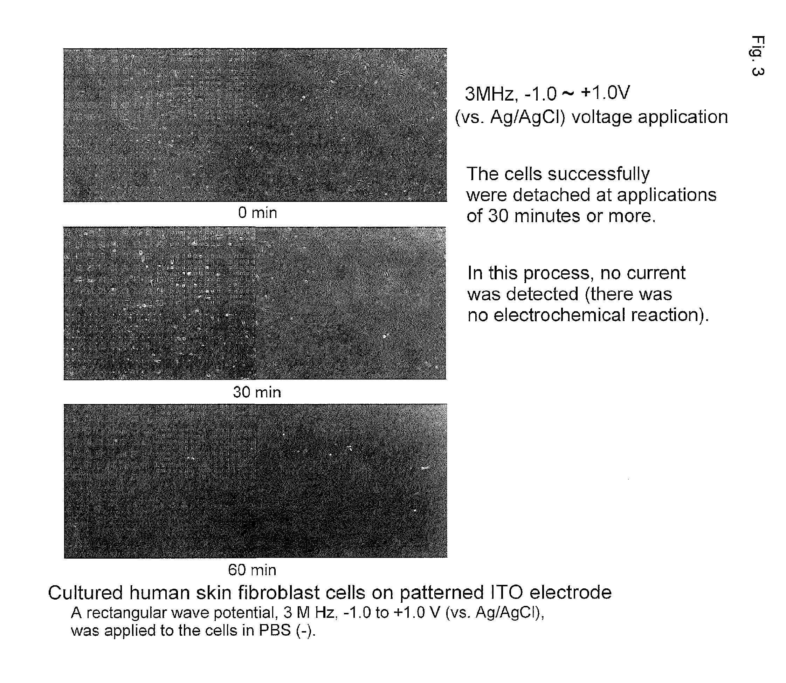

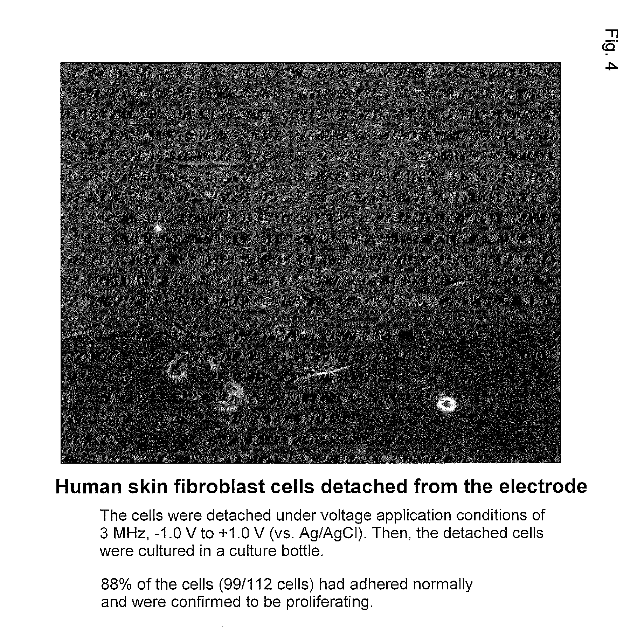

The results are given in Figure 3 in the form of images of voltage application for 0, 30, and 60 minutes. Under voltage application conditions of 3 MHz, -1.0 V to +1.0 V (vs. Ag/AgCl), the cells successfully were detached at applications of 30 minutes or more. In this process, no current was detected (there was no electrochemical reaction). When the cells were subcultured in a culture bottle following electric separation, as shown in Figure 4, 88% of the cells (99/112 cells) had adhered normally after four hours and were confirmed to be proliferating.

### Comparative Example 1

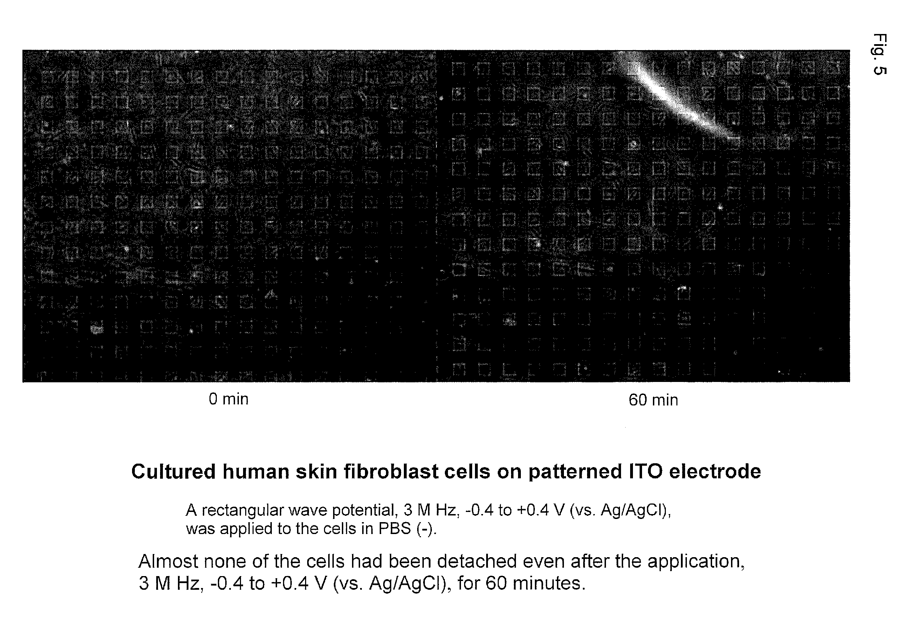

An attempt was made to detach the cells from the electrode under the same conditions as in Example 1 with the exception that the voltage application conditions were changed to 3 MHz, -0.4 to +0.4 V (vs. Ag/AgCl). Figure 5 shows the results in the form of images of voltage application for 0 and 60 minutes. Under these voltage application conditions, almost none of the cells had been detached even after application for 60 minutes.

### Comparative Example 2

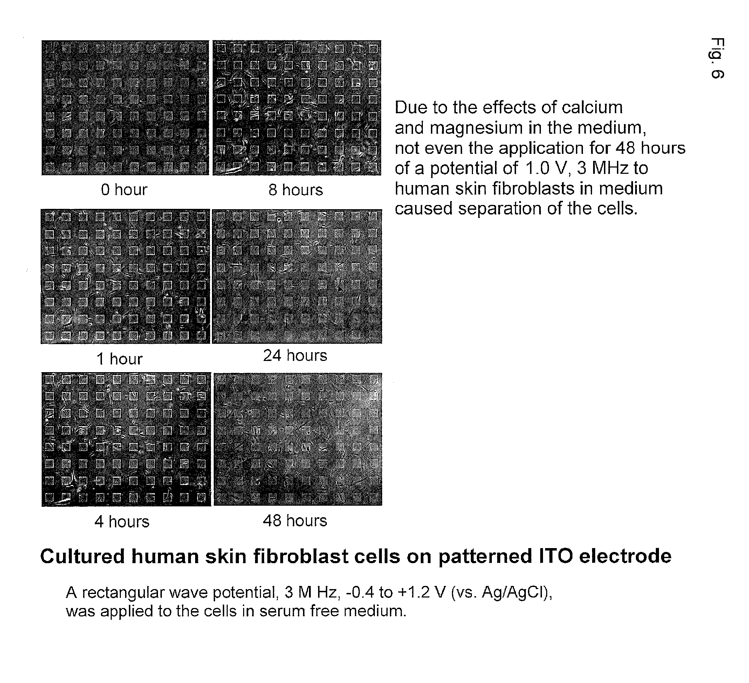

As shown in Figure 6, due to the effects of calcium and magnesium in the medium, not even the application for 48 hours of a potential of ±1.0 V, 3 MHz to human skin fibroblasts in medium caused separation of the cells.

### Comparative Example 3

Animal cells were detached by applying a constant potential by the following procedure using the three-electrode culture system prepared in Reference Example 1.

1) Animal cells (normal human skin fibroblasts) suspended in a serum-containing medium were inoculated into the ITO patterned electrode chamber and cultured for several days.
2) Attachment of the cells to the electrode substrate was confirmed and the interior of the ITO patterned electrode chamber was replaced with PBS(-).
3) A potentiostat (PS-14, Toho Technical Research) was used to apply a constant potential of -1.0 V (vs. Ag/AgCl) to the ITO patterned elected in the three-electrode culture system.
4) Observation was conducted by phase-contrast microscopy CKX-41, Olympus).
5) The live and dead animal cells were differentiated after applying a potential for 60 minutes in a 0.4% trypan blue in PBS(-) solution (ICN Biomedicals, Aurora, Ohio, USA).

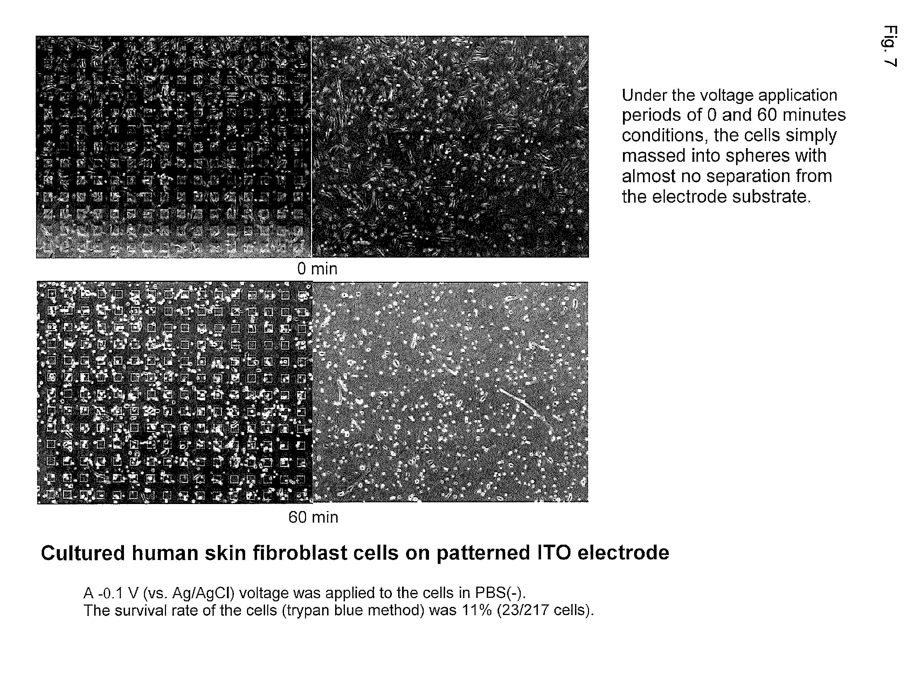

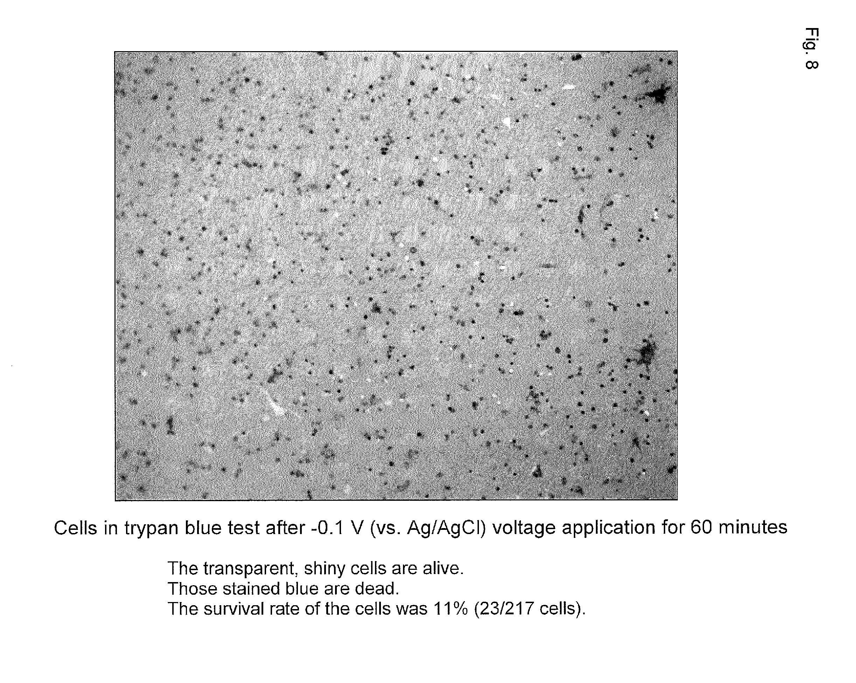

The results are given in Figure 7 in the form of images of voltage application periods of 0 and 60 minutes. Under these voltage application conditions, the cells simply massed into spheres with almost no separation from the electrode substrate. Figure 8 shows the results of differentiation with trypan blue. The transparent, shiny cells are alive. Those stained blue are dead. The survival rate of the cells was 11 % (23/217 cells).

### Example 2

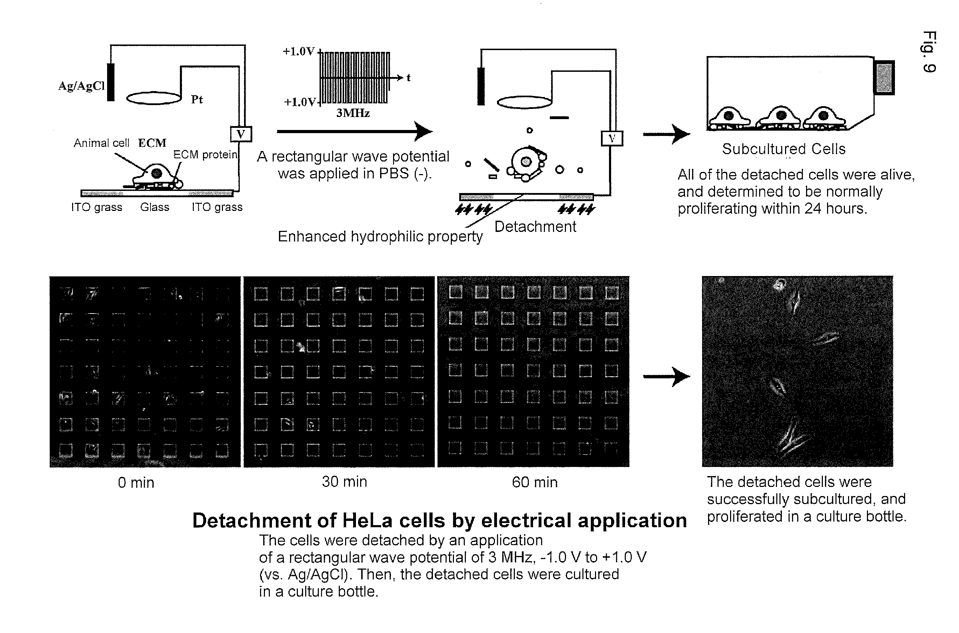

Cells were cultured and detached by the same methods as in Example 1 with the exception that an electrode substrate corresponding to (C) in Figure 1 was employed. Figure 9 shows images for voltage application periods of 0, 30, and 60 minutes. Under voltage application conditions of 3 MHz, -1.0 to +1.0 V (vs. Ag/AgCl), an application period of 30 minutes or more successfully caused the cells to be detached. In this process, no current was detected (there was no electrochemical reaction). When the cells were subcultured in a culture bottle following electric separation, the cells were determined to have adhered normally and to be proliferating within 24 hours.

### Reference Example 2

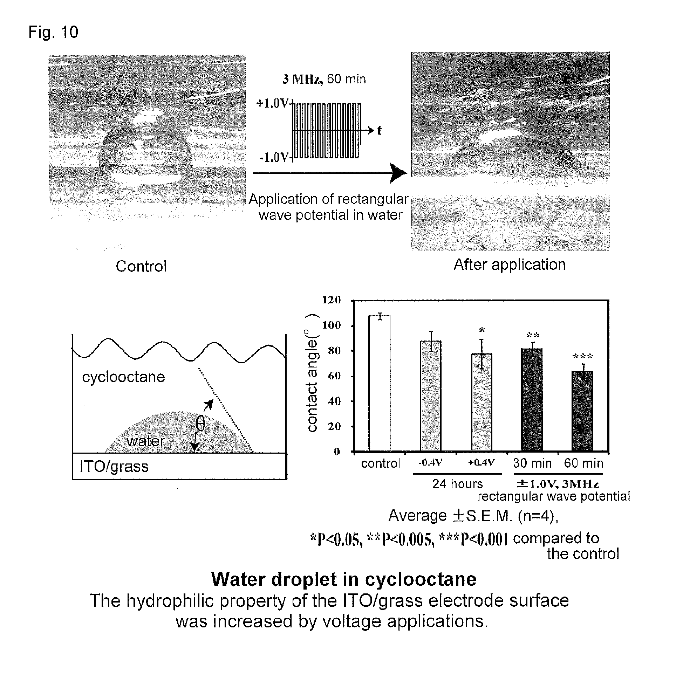

The water contact angle of the ITO electrode surface was measured when a constant voltage or high-frequency wave voltage was being applied to the ITO electrode. The measurement was conducted by placing a water droplet on the surface of an ITO electrode in an electrolytic bath filled with cyclooctane and measuring the contact angle before and after the application (24 hours) of a constant voltage (-0.4 V or +0.4 V (vs. Ag/AgCl)) or the contact angle before and after the application for 30 or 60 minutes of a high-frequency wave potential (±1.0 V, 3 MHz, rectangular waveform wave potential). The results are given in Figure 10. It will be understood that while the application of constant potentials (vs. Ag/AgCl) of -0.4 V and +0.4 V for 24 hours caused the water contact angle to decrease by about 20 to 30%, the application of a high-frequency wave potential for 30 or 60 minutes caused a drop of about 20 to 40 percent, quickly increasing the hydrophilic property of the electrode. Based on these results, it was presumed that the separation due to the application of a high-frequency wave potential to the animal cells in the present invention was due in part to the enhanced hydrophilic property of the electrode surface.

### [Industrial Applicability]

The present invention is useful in fields in which animal cells capable of proliferation are needed, such as in regenerative treatments.

## Claims

1. A method for preparing animal cells capable of proliferation, comprising the steps of
(1) culturing animal cells on a substrate surface at least one portion of which is an electrode, and
(2) applying a high-frequency wave potential to the electrode to detach the cells that have adhered to the substrate surface through culturing, wherein the high-frequency wave potential is of a frequency falling within a range of 1 KHz to 10 MHz and the range of the potential is ± 1.0 V (vs. Ag/AgCl) or less; and the culture medium during the detachment step is a culture liquid containing neither Ca²⁺ nor Mg²⁺.

2. The preparation method according to claim 1, wherein the culture medium during the detachment step is a phosphate buffer solution (containing neither Ca²⁺ nor Mg²⁺).

3. The preparation method according to claim 1 or 2, wherein the high-frequency wave potential is a rectangular wave, sinusoidal wave, or triangular wave.

4. The preparation method according to any one of claims 1 to 3, wherein the entire substrate surface is an electrode.

5. The preparation method according to any one of claims 1 to 3, wherein a portion of the substrate surface is an electrode and cells on the electrode surface and on a nonelectrode surface in proximity to the electrode are detached in step (2).

6. The preparation method according to any one of claims 1 to 5, further comprising the step of subculturing the animal cells capable of proliferation which have been detached to keep culturing animal cells that are capable of proliferation.

7. The preparation method according to any one of claims 1 to 6, wherein the cells are cultured in the form of a sheet, and the sheet of the cells is detached in a condition capable of proliferation to obtain a sheet of animal cells capable of proliferation.

8. The preparation method according to any one of claims 1 to 7, wherein the animal cells are skin cells.
